(19) Europäisches Patentamt
European Patent Office
Office européen des brevets

(11) **EP 2 652 510 B1**

(12) **EUROPÄISCHE PATENTSCHRIFT**

(45) Veröffentlichungstag und Bekanntmachung des Hinweises auf die Patenterteilung:
**22.06.2016 Patentblatt 2016/25**

(21) Anmeldenummer: **11793841.5**

(22) Anmeldetag: **13.12.2011**

(51) Int Cl.:
**G01N 33/66** (2006.01)     **G01N 33/543** (2006.01)
**G01N 33/53** (2006.01)

(86) Internationale Anmeldenummer:
**PCT/EP2011/072622**

(87) Internationale Veröffentlichungsnummer:
**WO 2012/080258 (21.06.2012 Gazette 2012/25)**

(54) **VERWENDUNG VON HYDROGELEN FÜR BIOSENSOREN MIT ERHÖHTER SENSITIVITÄT**

USE OF HYDROGELS FOR BIOSENSORS HAVING ELEVATED SENSITIVITY

UTILISATION D'HYDROGELS POUR DES BIOCAPTEURS À SENSIBILITÉ ACCRUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2010 EP 10195668**

(43) Veröffentlichungstag der Anmeldung:
**23.10.2013 Patentblatt 2013/43**

(73) Patentinhaber: **EyeSense AG**
**4051 Basel (CH)**

(72) Erfinder:
• **MÜLLER, Achim**
**63762 Großostheim (DE)**
• **HERBRECHTSMEIER, Peter**
**61462 Königstein (DE)**
• **KNUTH, Monika**
**63741 Aschaffenburg (DE)**
• **NIKOLAUS, Katharina**
**63739 Aschaffenburg (DE)**

(74) Vertreter: **Herzog, Fiesser & Partner**
**Patentanwälte PartG mbB**
**Patentanwälte**
**Dudenstrasse 46**
**68167 Mannheim (DE)**

(56) Entgegenhaltungen:
**EP-A1- 1 705 485        EP-A1- 2 052 677**
**WO-A1-01/16575         WO-A1-2006/061207**
**WO-A1-2007/065653      WO-A2-2008/142158**
**US-B1- 6 485 703**

• **CHEUNG ET AL: "Reusable optical bioassay platform with permeability-controlled hydrogel pads for selective saccharide detection", ANALYTICA CHIMICA ACTA, ELSEVIER, AMSTERDAM, NL, Bd. 607, Nr. 2, 3. Dezember 2007 (2007-12-03), Seiten 204-210, XP022413511, ISSN: 0003-2670, DOI: DOI:10.1016/J.ACA.2007.11.046**
• **Hans-Georg Elias: "Makromoleküle", 1990, Hüthig & Wepf Verlag, Basel ISBN: 3-85739-101-4 vol. 1, pages 680-681,**

**Beschreibung**

Die vorliegende Erfindung betrifft die Verwendung eines Polymer-basierten Hydrogels umfassend zumindest ein wasserlösliches Polymer in einem Sensor zum Anreichern eines gelösten Glukose-bindenden Proteins, wobei das zumindest eine Polymer in der Lage ist, mit dem Glukose-bindenden Protein zu interagieren und wobei das Glukose-bindende Protein in einem Komplex mit einem Liganden vorliegt.

Die Bestimmung der Konzentration von Glukose durch effiziente und zuverlässige Messtechnik ist in vielen Bereichen der Technik von großer Bedeutung. Nicht nur in der reinen Laboranalytik sondern auch in der Lebensmittelindustrie, z.B. im Bereich der Önologie, sowie im medizinischen Bereich, beispielsweise bei der Diagnose von Erkrankungen, die durch einen gestörten Glukosemetabolismus verursacht werden, z.B. Diabetes mellitus oder metabolisches Syndrom, spielt die schnelle und zuverlässige Bestimmung der Glukosekonzentration in vorgegebenen Lösungen eine zentrale Rolle. Im Bereich der Diagnose von Erkrankungen, die auf gestörtem Glukosemetabolismus basieren, werden Glukosesensoren sowohl in Vorrichtungen verwendet, die in den Körper implantierbar sind und den Glukosegehalt in einer innerhalb des Körpers genommenen Probe messen können, als auch in Sensoren, die ex vivo den Glukosegehalt aus einer Probanden Probe messen.

Zur Bestimmung des Glukosegehalts in einer Lösung sind Systeme aus Sensormolekül und Ligand beschrieben worden, wobei der Sensor ein Glukose-bindendes Protein und der Ligand ein Kompetitor für Glukose ist, der im Sensor zunächst am Glukose-bindenden Protein gebunden vorliegt. Durch Kompetition mit Glukose während des Messvorgangs wird der Kompetitor vom Glukose-bindenden Protein verdrängt. Die Verdrängung des Kompetitors durch die Glukose vom Glukose-bindenden Protein kann hierbei mittels einer Änderung von einer physikalischen oder chemischen Eigenschaft der Moleküle, z.B. mittels Fluoreszenz-Resonanz-Energietransfer (FRET), nachgewiesen werden. Die zuvor genannten Systeme müssen natürlich in einem räumlich begrenzten Bereich des Sensors vorliegen.

Zum Einschluss der Systemkomponenten, d.h. des Glukose-bindenden Proteins und des Liganden, in wässrigem Milieu werden semipermeable Membranen verwendet. Solche Membrane können beispielsweise aus regenerierter Zellulose, Polyethylenglykol, Polyurethan, layer-bylayer (LBL) Schichten, Polyethersulfonen, Parylen-Schichten oder perforiertem Silika bestehen (s. z.B. US2007/0122829). Alternativ kommen auch Hydrogele zum Einsatz, die aber üblicherweise auch von einer zusätzlichen Membran eingeschlossen werden (s. z.B. US 6,485,703; US2007/0105176).

Die im Stand der Technik beschriebenen Glukosesensoren weisen jedoch eine relativ geringe Glukoseaktivität auf. Die Glukoseaktivität und damit die Sensorleistung wird maßgeblich durch die Bindungskonstanten für die Komplexe aus Glukose-bindendem Protein und Kompetitor sowie Glukose-bindendem Protein und Glukose bestimmt. Zudem spielt auch die Beweglichkeit der Sensorkomponenten eine Rolle. Diese ist in Hydrogelmatrices eher eingeschränkt (Rounds 2007, J. Fluorec. 17: 57-63).

Die Publikation von Cheung et al. (Anal. Chim. Acta (2008), 607, S. 204-210) beschreibt die Entwicklung einer wiederverwendbaren Bioassay-Plattform unter Verwendung von permeabilitätsgesteuerten Hydrogelpads zum selektiven Nachweis von Sacchariden.

Bei in vivo Sensoren zur Messung von Glukose muss der Sensor ständig reversibel auf Konzentrationsänderungen des Analyten reagieren. Somit darf die Bindungskonstante nicht zu hoch sein, da der Sensor sonst schon bei niedrigen Analytkonzentrationen gesättigt wäre und Konzentrationsänderungen nicht mehr anzeigen könnte. Zudem besteht eine weitere Problematik der in vivo Sensoren darin, dass der Bereich der Analytkonzentration festliegt und nicht durch Verdünnengen oder Ankonzentrierungen optimiert werden kann. Im Stand der Technik werden im Wesentlichen Systeme aus Ligand und Glueose-bindendem Protein beschrieben, bei denen mittlere Bindungskonstanten verwirklicht werden. Eine Anpassung der Messempfindlichkeiten durch Änderung der Konzentrationen (z.B. durch Anreichern) von entweder Glukose-bindendem Protein oder Ligand oder beidem, sind regelmäßig durch die geringe Löslichkeit der entsprechenden Moleküle begrenzt. Insofern ist bei den im zuvor genannten Stand der Technik beschriebenen Sensoren sowohl die Messempfindlichkeit als auch die Messgenauigkeit des Sensors eingeschränkt.

Aufgabe der vorliegenden Erfindung ist es, Maßnahmen bereitzustellen, die es erlauben die Messempfindlichkeit und genauigkeit von Biosensoren zur Messung des Glukosespiegels zu verbessern. Die Erfindung wird durch die in den Ansprüchen beschriebenen Ausführungsformen sowie die Ausführungsformen, die anschließend offenbart werden, gelöst.

Die Erfindung betrifft somit die Verwendung eines Polymer-basierten Hydrogels bestehend aus zumindest einem wasserlöslichen Polymer in einem Sensor zum Anreichern eines gelösten Glukose-bindenden Proteins, wobei das zumindest eine Polymer in der Lage ist, mit dem Glukose-bindenden Protein zu interagieren und wobei das Glukose-bindende Protein in einem Komplex mit einem Liganden vorliegt, wobei das Polymer-basierte Hydrogel eine erste Hydrogelmatrix aus Alginat umfasst und eine zweite Hydrogelmatrix, die innerhalb der ersten Hydrogelmatrix ein interpenetrierendes Netzwerk ausbildet.

Der Begriff "Hydrogel" beschreibt ein Wasser-enthaltendes Polymer, dessen Moleküle chemisch oder physikalisch zu einem dreidimensionalen Netzwerk verknüpft sind. Die Polymermoleküle können untereinander durch kovalente oder ionische Bindungen oder durch Verschlaufen oder Verweben zu dem dreidimensionalen Netzwerk verknüpft werden. Die Polymere, die das Hydrogel bilden, enthalten hydrophile Polymerkomponenten, die die Aufnahme von wässrigen Lösungen ermöglichen sowie Gruppen, die in der Lage sind, mit dem Glukose-bindenden Protein zu interagieren. Insbesondere für Lektine wie Concanavalin A wurden neben einer Bindungsstelle für Monosaccharide, z.B. Glukose, auch eine weitere Bindungsstelle für endständige Zuckermoleküle beschrieben (Moothoo 1999, Glycobiology 9(6): 539-545; Bryce 2001, Biophysical Journal 81: 1373-1388; Moothoo 1998, Glycobiology 8(2): 173-181; Delatorre 2007, BMC Structural Biology 7:52). Zucker oder strukturell ähnliche Moleküle bzw. Reste in den Polymer-Molekülen des Hydrogels sind bevorzugt in der Lage mit dieser weiteren Bindungsstelle zu interagieren. Je nach Art des Glukose-bindenden Proteins wird erfindungsgemäß ein Polymer ausgewählt, welches über Reste verfügt, die in der Lage sind, mit dem Glukose-bindenden Protein zu interagieren ohne dabei die Bindungsstelle für Glukose zu blockieren. Geeignete Reste können hierbei auch Antikörper oder Antikörperfragmente sein, wie Fab F(ab)$_2$, scFv oder ähnliche, die an das Polymer gekoppelt werden und die das Glukose-bindende Protein spezifisch erkennen. Statt Antikörper können in analoger Weise auch Aptamere verwendet werden, die das Glukose-bindende Protein spezifisch erkennen.

Das zumindest eine Polymer ist ausgewählt aus der Gruppe bestehend aus: Alginaten, Sepharose, Hyaluronsäure, Chitosan, Caragenanen, Polyvinylalkoholen (PVAs), Polyethylenglykolen (PEGs), Poly(2-oxazolinen), Polyacrylamiden (z.B. Dimethylacrylamid), Polyhydroxyacrylaten (z.B. Polyhydroxymetacrylat, Polyhydroxyacrylamide, Polyvinylpyrolinone), (2-Methyl-3-Ethyl[2-Hydroxyethyl]) Polymeren, Polyhydroxyalkanoaten (PHAs), Poly (2-Methyl-2 oxazolinen), Poly (2-Ethyl-2 oxazolinen), Poly (2-Hydroxyethyl-2 oxazolinen), Poly (2-(1-(Hydroxymethyl)-ethyl)-2 oxazolinen), Poly-(hydroxyethylmetacrylat) (PHEMA), Poly-(hydroxyethylacrylat) (PHEA), Poly-vinylpyrolidonen, Poly-(dimethyl)acrylamid, Poly-(hydroxyethyl)acrylamid, Polyvinylalkoholen (einschließlich Copolymere mit Vinylacetate und/oder Ethylene), Poly(ethylene-co-vinylalkohol), Poly(vinylacetat-co- vinylalkohol), Poly(ethylene-co-vinylacetat-co-vinylalkohol), Polyethylenglycolen und Poly(ethylenglycol-co-propylenglycol).

Bevorzugt ist das zumindest eine Polymer ausgewählt aus der Gruppe bestehend aus:

Alginaten, Sepharose, Hyaluronsäure, Chitosan, und Caragenanen. Ganz besonders bevorzugt ist das zumindest eine Polymer ein Alginat.

Es versteht sich, dass das Hydrogel im Sensor noch weitere Polymere aufweisen kann. Insbesondere kann das Polymer-basierte Hydrogel eine erste Hydrogelmatrix aus Alginat und eine zweite Hydrogelmatrix die innerhalb der ersten Hydrogelmatrix ein interpenetrierendes Netzwerk ausbildet, umfassen. Diese zweite Hydrogelmatrix besteht vorzugsweise aus einem wasserlöslichen Polymer mit mindestens einer vernetzbaren Gruppe pro Molekül und einem Molekulargewicht von höchstens 500.000. Besonders bevorzugst ist ein Molekulargewicht von höchstens 250.000, 200.000, 150.000, 100.000 oder 50.000. Bevorzugt ist das Polymer der zweiten Hydrogelmatrix hierbei ausgewählt aus der Gruppe bestehend aus: Polyvinylalkoholen (PVAs), Polyethylenglykolen (PEGs), Poly(2-oxazolinen), Polyacrylamiden (z.B. Dimethylacrylamid), Polyhydroxyacrylaten (z.B. Polyhydroxymetacrylat, Polyhydroxyacrylamide, Polyvinylpyrolinone), (2-Metliyl-3-Ethyl[2-Hydroxyethyl]) Polymeren, Polyhydroxyalkanoaten (PHAs), Poly (2-Methyl-2 oxazolinen), Poly (2-Ethyl-2 oxazolinen), Poly (2-Hydroxyethyl-2 oxazolinen), Poly (2-(1-(Hydroxymethyl)-ethyl)-2 oxazolinen), Poly-(hydroxyethylmetalcrylat) (PHEMA), Poly-(hydroxyethylacrylat) (PHEA), Poly-vinylpyrolidonen, Poly-(dimethyl)acrylamid, Poly-(hydroxyethyl)acrylamid, Polyvinylalkoholen ((einschließlich Copolymere mit Vinylacetate und/oder Ethylene), Poly(ethylene-co-vinylalkohol), Poly(vinylacetat-co-vinylalkohol), Poly(ethylene-co-vinylacetat-co-vinylalkohol-), Polyethylenglycolen und Poly(ethylenglycol-co-propylenglycol).

Besonders bevorzugt wird die zweite Hydrogelmatrix aus Polyvinylalkohol gebildet. Besonders bevorzugt wird der Polyvinylalkohol mit einem Molekulargewicht von 10.000 bis 100.000, mehr bevorzugt 10.000 bis 50.000, weiterhin bevorzugt 10.000 bis 20.000 und ganz besonders bevorzugt 15.000. Besonders bevorzugt weist der Polyvinylalkohol einen Vernetzergehalt von höchsten 0,5 mmol/g, 0,4 mmol/g, 0,35 mmol/g oder 0,3 mmol/g und ganz besonders bevorzugt 0,35 mmol/g auf. Weiterhin weist der Polyvinylalkohol besonders bevorzugt einen Feststoffgehalt des Präpolymers von weniger als 40 Gewichtsprozent auf.

Das erfindungsgemäße Hydrogel kann neben der ersten Hydrogelmatrix und der zweiten Hydrogelmatrix Zusatzstoffe enthalten, z.B. Stabilisatoren, Emulgatoren, Antioxidantien, UV-Stabilisatoren, Detergentien, und/oder UV-Initiatoren.

Das erfindungsgemäß verwendete Hydrogel kann zudem von einem weiteren, bevorzugt semipermeablen, Hüllmaterial umschlossen sein. Durch diese Umschließung wird ein "Ausbluten" ("leaching") der Sensorkomponenten aus dem Hydrogel verhindert. Als Hüllmaterial kommen semipermeable Membranen oder weitere Hydrogelmatrices in Frage. Semipermeable Membranen können bevorzugt aus regenerierter Zellulose, Polyethylenglykol, Polyurethan, layerbylayer (LBL) Schichten, Polyethersulfonen, Parylen-Schichten oder perforiertem Silika bestehen. Als Hüllmaterial kommt aber auch eine weitere Hydrogelmatrix in Frage. Bevorzugt kann diese aus einem Polymer gebildet werden ausgewählt aus der Gruppe bestehend aus Alginaten, Sepharose, Hyaluronsäure, Chitosan, Caragenanen, Polyvinylalkoholen (PVAs), Polyethylenglykolen (PEGs), Poly(2-oxazolinen), Polyacrylamiden (z.B. Dimethylacrylamid), Polyhydroxyacrylaten (z.B. Polyhydroxymetacrylat, Polyhydroxyacrylamide, Polyvinylpyrolinone), (2-Methyl-3-Ethyl[2-Hydroxyethyl]) Polymeren, Polyhydroxyalkanoaten (PHAs), Poly (2-Methyl-2 oxazolinen), Poly (2-Ethyl-2 oxazolinen), Poly (2-Hydroxyethyl-2 oxazolinen), Poly (2-(1-(Hydroxymethyl)-ethyl)-2 oxazolinen), Poly-(hydroxyethylmetacrylat) (PHEMA), Poly-(hydroxyethylacrylat) (PHEA), Poly-vinylpyrolidonen, Poly-(dimethyl)acrylamid, Poly-(hydroxyethyl)acrylamid, Polyvinylalkoholen (einschließlich Copolymere mit Vinylacetate und/oder Ethylene), Poly(ethylene-co-vinylalkohol), Poly(vinylacetat-co-vinylalkohol), Poly(ethylene-co-vinylacetat-co-vinylalkohol), Polyethylenglycolen und Poly(ethylenglycol-co-propylenglycol).

Der Begriff "Glukose-bindendes Protein" bezieht sich im Rahmen der Erfindung auf Proteine, die in der Lage sind, spezifisch mit Glukose zu interagieren. Ob ein Protein in der Lage ist, spezifisch mit Glukose zu interagieren, kann durch im Stand der Technik bekannte Bindungstests vom Fachmann ohne weiteres bestimmt werden. Bevorzugt ist das Glukose-bindende Protein zudem in der Lage, mit dem Polymer des Hydrogels zu interagieren. Bevorzugt wird diese Interaktion nicht durch die Glukose Bindungsstelle des Glukose-bindenden Proteins vermittelt. Besonders bevorzugt ist das Glukose-bindende Protein, ausgewählt aus der Gruppe bestehende aus Lektinen, Enzymen, die Glukose als Substrat binden, und Antikörpern, die spezifisch Glukose erkennen. Umfasst von dem Begriff sind auch Surrogatmoleküle, die Glukose spezifisch erkennen können, bevorzugt Aptamere, die spezifisch Glukose erkennen. Ganz besonders bevorzugt ist das Glukose-bindende Protein Concanavalin A.

Nukleinsäuresequenzen und Aminosäuresequenzen, die die zuvor genannten Glukose-bindenden Proteine codieren, sind im Stand der Technik bekannt (Yamauchi 1990, FEBS Letters 260(1): 127-130). Dementsprechend können die zuvor genannten Proteine ohne weiteres vom Fachmann bereitgestellt werden. Die besagten Proteine können beispielsweise rekombinant hergestellt werden oder aus einer biologischen Quelle aufgereinigt werden. Darüber hinaus können die Proteine auch chemisch synthetisiert werden. Die meisten der zuvor genannten Proteine sind zudem kommerziell erhältlich. Antikörper oder Aptamere, die spezifisch Glukose erkennen, können vom Fachmann ohne weiteres durch im Stand der Technik bekannte Methoden zur Antikörper- oder Aptamer-Gewinnung bereitgestellt werden.

Die zuvor genannten Glukose-bindenden Proteine und insbesondere Concanavalin A können bevorzugt auch chemische Modifikationen aufweisen, die eine im Vergleich zu unmodifizierten Versionen der Glukose-bindenden Proteine erhöhte Wasserlöslichkeit vermitteln. Solche Modifikationen umfassen bevorzugt die Funktionalisierung mit einem wasserlöslichen Polymer und können in einer besonders bevorzugten Ausführungsform ausgewählt werden, aus der Gruppe bestehend aus: Pegylierung, Acetylierung Polyoxazolinylierung und Succinylierung.

Der Nachweis von Glukose in einer zu analysierenden Probe erfolgt in der erfindungsgemäßen Vorrichtung durch eine Verdrängung des an das Glukose-bindende Protein gebundenen Liganden durch die in der Probe enthaltene Glukose (Kompetition zwischen Ligand und Glukose). Bevorzugt hat der Ligand daher eine geringere Affinität zu dem Glukose-bindenden Protein als die Glukose. Die Verdrängung kann bevorzugt nachgewiesen werden durch Markierung des Liganden mit einem Farbstoff oder einem anderen nachweisbaren Markermolekül. Als besonders geeignet für den Nachweis einer Verdrängung haben sich Farbstoffe oder andere Markermoleküle erwiesen, die bei Annäherung der damit verbundenen Moleküle eine Änderung von zumindest einer messbaren physikalischen oder chemischen Eigenschaft hervorrufen. Geeignete Systeme umfassen solche, bei denen mittels Energietransfer zwischen den Farbstoffmolekülen entweder ein messbares Signal unterdrückt oder generiert wird. Solche auf Energietransfer basierte Systeme sind beispielsweise in WO2001/13783 näher beschrieben. Bevorzugt kann es sich hierbei um Systeme handeln bei denen durch Quenching Effekte ein Fluoreszenz Signal unterdrückt wird, wenn die Farbstoff oder Markermoleküle - und damit das Glukose-bindende Protein und sein Ligand - in räumlicher Nähe sind. Nach der Verdrängung des Liganden durch den Analyten wird der Quenching Effekt dann aufgehoben. Dieser Effekt ist durch eine Veränderung der Fluoreszenz nachweisbar. Zum Nachweis können beispielsweise Fluoreszenz-Photometer wie in WO2002/087429 beschrieben eingesetzt werden. Weitere geeignete System sind sogenannte Fluoreszenz-Resonanz-Energietransfer- (FRET) basierte Nachweissysteme. Hierbei werden zwei interagierende Komponenten, wie das Glucose-bindende Protein und sein Ligand, mit Fluoreszenz-Farbstoffen markiert. Eine Komponente wird mit einem Akzeptor-Farbstoff, die andere mit einem Donor-Farbstoff gekoppelt. Durch die Interaktion der Komponeneten kommen die Farbstoffe in räumliche Nähe wodurch der FRET Effekt vermittelt wird. Bei diesem wird Anregungsenergie vom Donor- auf den Akzeptor-Farbstoff transferiert und somit die Intensität des Donor-Farbstoffes messbar verringert. Sobald die Interaktion der Komponenten unterbrochen wird, nimmt die Fluoreszenz Intensität des Donors wieder zu. Im Fall der erfindungsgemäßen Vorrichtung kann die Glukose somit über die Zunahme der Intensität eines Signals nachgewiesen werden, das von einem Farbstoff oder Markermolekül nach Trennung des Komplexes aus Glukose bindendem Protein und Ligand durch den Analyten generiert wird. Der Farbstoff oder das Markermolekül ist entweder an das Glukose-bindenden Protein oder den Liganden gekoppelt. Beispielsweise kann ein Donor Farbstoff an das Glukose-bindenden Protein oder den Liganden gekoppelt sein, wobei die nicht mit dem Donor- Farbstoff gekoppelte Komponente mit einem geeigneten Akzeptor-Farbstoff gekoppelt ist. In einer derart ausgestalteten erfindungsgemäßen Vorrichtung ist vor Beaufschlagung mit einer Glukosehaltigen Probe der FRET Effekt als Resultat der Bindung des Liganden an das Glukose-bindende Protein zu beobachten. Nach Beaufschlagung verdrängt die Glukose den Liganden, so dass die messbare Intensität der Fluoreszenz des Donor-Farbstoffes zunimmt und zwar proportional zur Menge an Glukose.

Birch et. al. (Birch 2001, Spectrochimica Acta Part A 57: 2245-2254) haben die mathematische Lösung des chemischen Gleichgewichts für den Fall Concanavalin A als Glukose-bindendes Protein und Dextran als Ligand berechnet. Simulationen mit variierenden Concanavalin A und Dextran Konzentrationen haben ergeben, dass das Verhältnis (Dextran) / (ConA-Dextran-Komplex) nur unwesentlich von den Ausgangskonzentrationen abhängt und die Bindungskonstanten $K_{Dex}$ und $K_{Gluc}$ die Hauptfaktoren für die Sensor-Performance sind.

Überraschenderweise wirkt sich die Struktur der Farbstoffe jedoch ebenfalls auf die Glukose-Aktivität aus. So ist erfindungsgemäß eine Kombination aus einem Rhodamin- und einem Oxazin-Farbstoff der herkömmlich verwendeten Kombination aus einem Xanthen- und einem Rhodamin-Farbstoff (FITC-TMR) deutlich überlegen.

Bevorzugt ist das Glukose-bindende Protein, das im Rahmen der erfindungsgemäßen Verwendung eingesetzt wird, mit einem Oxazin-Farbstoff verknüpft. Wie eine solche Verknüpfung erfolgen kann, ist dem Fachmann wohl bekannt und im Stand der Technik hinreichend beschrieben. Besonders bevorzugt ist der Oxazin-Farbstoff, ein Oxazin-Akzeptor, ausgewählt aus der Gruppe bestehend aus: ATTO655, ATTO680, EVOblue10, EVOblue30, EVOblue90 und EVOblue100. Ganz besonders bevorzugt wird ATTO680 eingesetzt. Die genannten Oxazin-Farbstoffe sind kommerziell erhältlich.

Bevorzugter Markierungsgrad (DOL, "degree of labeling") für das Glukose-bindende Protein, z.B. Concanavalin A, ist 0,1 bis 4, stärker bevorzugt 1 bis 4 und besonders bevorzugt 1 bis 3. Bei Concanavalin A entspricht ein DOL von 1 hierbei einem Mol Farbstoff pro Mol Concanavalin A Tetramer (MW = 104.000). Bei hohem DOL und Verwendung von relativ unpolaren Farbstoffen (typischerweise langwellige Fluoreszenzfarbstoffe) ist das Glukose-bindende Protein vorzugsweise mit PEG funktionalisiert. Bevorzugter Pegylierungsgrad ist 0,1 bis 5, bevorzugtes Molekulargewicht ist 200 bis 10.000, besonders bevorzugt 800 bis 8.000 und ganz besonders bevorzugt 800 bis 5.000.

Im Rahmen der der vorliegenden Erfindung zugrunde liegenden Experimente wurde festgestellt, dass die chemischen Modifikationen der Glukose-bindenden Proteine, die eine im Vergleich zu unmodifizierten Varianten erhöhte Wasserlöslichkeit vermitteln, in vorteilhafter Weise eingesetzt werden können, um einen besseren Markierungsgrad an den Glukose-bindenden Proteinen zu erzielen. Für die modifizierten Glukose-bindenden Proteine gemäß der vorliegenden Erfindung können auch höhere Markierungsgrade mit einem Farbstoff erreicht werden als für unmodifizierte Varianten davon. Für solche modifizierten Concanavalin A Proteine können bevorzugt Konzentrationen größer als 0,5 mg/(g Matrix) und ganz besonders bevorzugt zwischen 2 und 60 mg/(g Matrix) erreicht werden. Überraschenderweise war die gemessene Glukoseaktivität von modifizierten Concanavalin A Proteinen hierbei auch vergleichbar mit der von nativem Concanavalin A in Hydrogel.

Bevorzugt kann das gelöste Glukose-bindende Protein im Hydrogel hierbei in einer im Vergleich zu einer wässrigen Lösung mindestens 5-fach, mindestens 10-fach, mindestens 15-fach oder mindestens 20-fach erhöhten Konzentration vorliegen.

Die verbesserte Löslichkeit ist insbesondere relevant wenn die Glukose-bindende Proteine mit einem Farbstoff markiert werden sollen, da mit Farbstoffen markierte Glukose-bindende Proteine, beispielsweise ein mit einem der zuvor genannten Oxazin-Farbstoffen modifiziertes Concanavalin A, eine in wässriger Lösung nochmals reduzierte Löslichkeit besitzen. Je höher hierbei der Markierungsgrad ist, desto niedriger ist die Löslichkeit in wässriger Lösung. Ein hoher Markierungsgrad wird jedoch gerade für Glukose-bindende Proteine als Sensorkomponenten in den erfindungsgemäßen Vorrichtungen benötigt.

Der Begriff "Ligand des Glukose-bindenden Proteins" bezeichnet ein Molekül, das in der Lage ist, eine spezifische Bindung mit dem Glukose-bindenden Protein einzugehen, wobei das Molekül im Wesentlichen mit der selben Bindungsstelle wie Glukose interagiert, so dass das gebundene Molekül durch Glukose von der Bindungsstelle am Glukose-bindenden Protein verdrängt werden kann. Als Ligand geeignete Moleküle sind daher strukturell mit Glukose verwandt. Vorzugsweise ist der Ligand des Glukose-bindenden Proteins ein Oligosaccharid, ein glykosyliertes Makromolekül, z.B. ein glykosyliertes Protein oder Peptid, oder ein glykosylierter Nanopartikel. Die zuvor genannten Moleküle, die als Liganden des Glukose-bindenden Proteins zum Einsatz kommen können, sind im Stand der Technik bekannt und können vom Fachmann ohne weiteres bereitgestellt werden. Besonders bevorzugt wird als Ligand des Glukose-bindenden Proteins ein Dextran eingesetzt.

Vorzugsweise ist der Ligand des Glukose-bindenden Proteins im Sensor mit einem Rhodamin-Farbstoff gekoppelt. Besonders bevorzugt können hierbei ATTO590, ATTO610, ROX, TMR, Rhodamin G6, Alexa Fluor Rhodamine Farbstoffe oder Dy590 und ganz besonders bevorzugt ATTO590 eingesetzt werden.

Bevorzugter Markierungsgrad (DOL) für den Liganden des Glukose-bindenden Proteins, z.B. Dextran, ist 0,00003 bis 0,017 (mol Farbstoff)/(mol Untereinheit), besonders bevorzugt 0,0005 bis 0,007 (mol Farbstoff)/(mol Untereinheit) und ganz besonders bevorzugt 0,001 bis 0,0016 (mol Farbstoff)/(mol Untereinheit). Der Markierungsgrad des Liganden hat ebenfalls einen Einfluss auf die Glucose-Aktivität. Zu geringe genauso wie zu hohe Markierungsgrade führen zu einer schlechteren Glukoseaktivität.

In einer bevorzugten Ausführungsform der erfindungsgemäßen Verwendung ist das Glucose-bindende Protein Concanavalin A. Ebenso weist das Concanavalin A durch chemische Modifikationen, bevorzugt Pegylierung, Acetylierung Polyoxazolinylierung oder Succinylierung, ein im Vergleich zum unmodifizierten Concanavalin A erhöhte Wasserlöslichkeit auf. Das Glukose-bindende Protein ist in einer bevorzugten Ausführungsform mit einem Oxazin-Farbstoff verknüpft und der Ligand des Glukose-bindenden Proteins mit einem Rhodamin-Farbstoff. Ganz besonders bevorzugt wird ein Concanavalin A / Dextran-System in der Sensor Vorrichtung verwendet, bei dem das Dextran mit einem Rhodamin-Donor Farbstoff und das Concanavalin A mit einem Oxazin-Akzeptor Farbstoff verknüpft ist. In dem bevorzugten Concanavalin A / Dextran System liegen die Komponenten bevorzugt in einem Massenverhältnisse (Dextran/ConA) von 1:1 bis 1:40 vor, wobei Massenverhältnisse nahe 1:10 besonders bevorzugt sind.

Es wurde im Rahmen der vorliegenden Erfindung festgestellt, dass die Kombination von Oxazin- und Rhodamin-Farbstoffen eine heterodimere Wechselwirkung zwischen den Farbstoffresten bewirkt, die die Glukoseaktivität verstärkt. Somit kann durch die Verwendung von Oxazin-Akzeptor und Rhodamin-Donor Farbstoffen bereits in wässriger Lösung vorzugsweise eine 2,1-fache Glukoseaktivität erzielt werden. In dem im Rahmen der vorliegenden Erfindung eingesetzten Hydrogel konnte sogar eine 2,6-fach Erhöhung der Glukoseaktivität bewirkt werden.

Durch die Verwendung eines Polymers, das in der Lage ist, mit dem Glukose-bindenden Protein zu interagieren, kann vorteilhafterweise eine höhere Konzentration von Glukose-bindendem Protein und Ligand im Hydrogel des Sensors erzielt werden. Dies erhöht die Messempfindlichkeit aber auch die Messgenauigkeit des Sensors, insbesondere bei in vivo Anwendungen mit gegebener Glukosekonzentration.

Im Rahmen der vorliegenden Erfindung wurde vorteilhafterweise festgestellt, dass die Verwendung eines Hydrogels, bestehend aus einer ersten Hydrogelmatrix als Alginat und einer zweiten Hydrogelmatrix, die innerhalb der ersten ein interpenetrierendes Netzwerk ausbildet, in der Lage ist, eine Umgebung für die Sensorkomponenten, nämlich das Glukose-bindenden Protein und den kompetitiven Liganden des Glukose-bindenden Proteins, zu schaffen, die eine effiziente Bestimmung der Glukoseaktivität erlaubt. Viele Ansätze zur Glukosemessung mittels Fluoreszenz sind in Lösung erfolgreich, verlieren jedoch ihre Aktivität, wenn die Sensorkomponenten in Hydrogelmatrices eingebettet werden, da die Beweglichkeit der Sensorkomponenten eingeschränkt wird (Rounds 2007, J. Fluoresc. 17: 57-63; US 2007/0105176 A1). Überraschenderweise insbesondere auch im Hinblick auf die Berechnungen von Birch et al (Birch 2001, loc cit) konnten jedoch im vorliegenden Fall eine gegenüber wässrigen Lösungen um bis zu 2,6-fach gesteigerte Aktivitäten festgestellt werden. Durch die Auswahl von geeigneten Hydrogelmatrices konnte eine Anreicherung des Glukose-bindenden Proteins weit über dessen Löslichkeitsgrenze in wässriger Lösung erzielt werden. Im Falle von Concanavalin A konnte beispielsweise eine mehr als 10-fache erhöhte Konzentration gegenüber einer in freier Lösung erreichbaren Konzentration erreicht werden. Üblicherweise wird die Löslichkeit der Rezeptorkomponente in freier Lösung noch negativ durch die Zugabe des Liganden beeinflusst, da der Rezeptor / Kompetitor-Komplex aufgrund seiner Größe und häufig auch aufgrund von Multivalenzen eine geringere Löslichkeit aufweist. Während beispielsweise ein Concanavalin A / Dextran-Komplex in Lösung bei einem Massenverhältnis von 1:10 schon ab einer Concanavalin A-Konzentration von 0,5 mg/(g Lösung) beginnt auszufallen, können in einem geeigneten Hydrogel bei gleichem Massenverhältnis Concanavalin A-Konzentration von über 50 mg/(g Matrix) eingestellt werden. Somit kann der anwendbare Konzentrationsbereich um ein 100-faches erweitert werden. Dies ist insbesondere von Bedeutung für Anwendungen, bei denen die Analytkonzentration festliegt und nicht durch Verdünnungen oder Ankonzentrierung angepasst werden kann. Solche Schwierigkeiten treten gerade bei in vivo Anwendungen, wie der Bestimmung des Glukosespiegels in Körperflüssigkeiten, auf. Die Konzentration des Analyten Glukose ist in der in vivo Situation nicht an die spezifischen Assay-Bedingungen anzupassen, sondern muss als gegeben hingenommen werden.

Ein weiteres Löslichkeitsproblem tritt insbesondere bei in vivo Anwendungen von biologischen Sensoren auf. Aufgrund der höheren Wellenlänge nimmt die Eigenfluoreszenz des Gewebes ab, so dass bei in vivo Anwendungen langwellige Fluoreszenz-Farbstoffe eingesetzt werden. Diese Fluoreszenz-Farbstoffe sind jedoch typischerweise aufgrund ihrer molekularen Struktur und Größe (konjungierte Systeme) apolar. Wird das Glukose-bindende Protein nur mit einem solchen Farbstoff markiert, erniedrigt sich die Löslichkeit zusätzlich, so dass auch keine hohen Markierungsgrade möglich sind. Wie bereits zuvor beschrieben, kann es daher notwendig werden, das Glukose-bindende Protein mit z.B. Polyethylenglykol zu funktionalisieren, um eine gesteigerte Löslichkeit und damit verbunden höhere Markierungsgrade zu ermöglichen. Die Funktionalisierung mit Polyethylenglykol (Pegylierung) führt jedoch in Lösung regelmäßig zu einer stark reduzierten Glukoseaktivität von zum Beispiel nativem Concanavalin A (relative Glukoseaktivität von 0,4 oder weniger). Überraschenderweise wurde im Rahmen der vorliegenden Erfindung festgestellt, dass in dem Hydrogel der Sensor Vorrichtung diese Verschlechterung nicht auftritt. Vielmehr können Glukoseaktivitäten für mit Polyethylenglykol funktionalisiertem Concanavalin A erzielt werden, die mit denen von nativem Concanavalin A vergleichbar sind (relative Glukoseaktivität = 2,2 bzw. 2,6). Darüber hinaus wurde gefunden, dass die Glukose-Aktivität durch die Erhöhung des Markierungsgrad am Concanavalin A noch zusätzlich gesteigert werden kann in einem Hydrogel, wie es in der Sensor Vorrichtung erfindungsgemäß eingesetzt wird. So wurden überraschenderweise in dem anreichenden Hydrogel durch Erhöhung des Markierungsgrads am mit Polyethylenglykol funktionalisierten Concanavalin A sogar eine Verdopplung der Glukoseaktivität erreicht. Im Vergleich zur Messung in Lösung wird die Glukoseaktivität in dem erfindungsgemäß verwendeten Hydrogel in der Sensor Vorrichtung sogar um das 4,3-fache gesteigert. Die Verwendung des Hydrogels in der erfindungsgemäßen Vorrichtung erlaubt es also, Konzentrationsverhältnisse für das Glukose-bindende Protein und seinen Liganden einzustellen, die bei entsprechenden Markierungsgraden Glukose-Aktivitäten erlauben, die um das 4-fache erhöht sind gegenüber von Konzentrationen, die in wässrigen Lösungen einstellbar sind. Dies ermöglicht vorteilhafter Weise auch den Einsatz der erfindungsgemäßen Vorrichtung unter Bedingungen, bei denen die Analytkonzentration nicht den Assay-Bedingungen angepasst werden kann.

Im Rahmen der erfindungsgemäßen Verwendung kann der Sensor bevorzugt zur Bestimmung des Glukosegehalts eingesetzt wird.

Hierbei kann eine Probe untersucht werden. Unter dem Begriff "Probe" ist im Rahmen der vorliegenden Erfindung eine Zusammensetzung, vorzugsweise eine wässrige Zusammensetzung, zu verstehen, die vermutlich oder tatsächlich Glukose enthält. Bevorzugt handelt es sich bei der Probe um eine biologische Probe. Ganz besonders bevorzugt handelt es sich bei der Probe um eine Körperflüssigkeit, insbesondere, Gewebeflüssigkeit (interstitielle Flüssigkeit), Blut, Plasma, Serum, Lymphe, Speichel, Tränenflüssigkeit, Schweiß oder Urin. Besonders bevorzugt handelt es sich bei der Probe um Gewebeflüssigkeit, Blut, Serum oder Plasma.

Sofern die Probe ein biologisches Material, z.B. eine Körperflüssigkeit ist, kann sie vorzugsweise aus einem Probanden gewonnen werden, der einen gestörten Glukosemetabolismus entweder tatsächlich oder vermutlich aufweist. Vorzugsweise wird der gestörte Glukosemetabolismus hierbei durch Diabetes mellitus oder das metabolische Syndrom verursacht.

Der Sensor kann im Rahmen der erfindungsgemäßen Verwendung also auch für die ex vivo Diagnostik von Erkrankungen oder Störungen des Glukose-Metabolismus eingesetzt werden. Insbesondere kann der Sensor zur Diagnose eines gestörten Glukosemetabolismus und damit verbunden von Erkrankungen wie Diabetes mellitus oder dem metabolischen Syndrom eingesetzt werden oder zur Bestimmung der Notwendigkeit einer therapeutischen Maßnahme bei einem Patienten mit gestörtem Glukosemetabolismus.

Die zuvor erwähnten therapeutischen Maßnahmen umfassen solche, die zur Behandlung von Diabetes mellitus oder dem metabolischen Syndrom eingesetzt werden. Hierzu gehört neben der Verabreichung von Arzneimitteln, z.B. Insulin, auch die Durchführung anderer therapeutischer Maßnahmen, über die aufgrund des ermittelten gestörten Glukose Metabolismus entschieden werden kann. Hierzu zählen therapeutische Eingriffe, z.B. Gastric-Bypass Operationen, oder Änderungen der Lebensgewohnheiten, z.B. die Durchführung spezieller Diäten. Der Sensor kann mit einer weiteren Vorrichtung gekoppelt werden, z.B. einer Vorrichtung, die die Abgabe eines Arzneimittels steuert. Bevorzugt ist hierbei eine Vorrichtung zur Abgabe von Insulin. Die Abgabe von Insulin kann dann basierend auf der durch den Sensor ermittelten Notwendigkeit gesteuert werden.

Der Sensor aus der erfindungsgemäßen Verwendung kann für die ex vivo Anwendung beispielsweise in Mikrotiterplatten eingebracht und dort verankert werden. Zu messende Proben werden dann in die Vertiefungen der Mikrotiterplatten aufgetragen und können dann mit einer Reader-Vorrichtung vermessen. Ein solcher Ansatz erlaubt das simultane Vermessen einer großen Zahl von Proben und ist somit auch kostengünstig, insbesondere im klinischen Diagnostik-Betrieb.

Beschrieben ist eine in vivo Anwendung. Hierzu kann der Sensor in den Körper eingebracht werden. Hierbei ist zu beachten, dass die Messung des Glukosespiegels, die ja auch die Basis für die Diagnose ist, voraussetzt, dass der Sensor in Kontakt mit einer Körperflüssigkeit kommt, die Glukose enthält, wobei die Konzentration der Glukose in der Flüssigkeit repräsentativ für den zu ermittelnden Glukosespiegel ist. Geeignete Körperflüssigkeiten werden an anderer Stelle in der Beschreibung aufgezählt. Besonders bevorzugt ist die Körperflüssigkeit aber Gewebeflüssigkeit.

Der Sensor wird bevorzugt an Stellen im Körper eingebracht, die eine optische Messung des vom Sensor generierten Signals ermöglichen. Geeignet sind Stellen mit entweder einer geringen Gewebedicke zwischen Vorrichtung und Körperoberfläche oder mit transparenten Geweben, die gut von dem generierten Signal durchdrungen werden können. Besonders bevorzugt wird die Vorrichtung unter der Haut (subkutan) oder im Auge (subkonjunktival) platziert. Entsprechende Verfahren zur Implantation der Vorrichtung sind im Stand der Technik bekannt.

Alternativ kann das von der Vorrichtung erzeugte Signal auch mittels eines geeigneten Übertragungsmediums aus dem Körper heraus übertragen werden. Hierzu kann vorzugsweise ein Signalleitendes Material als flexibles Kabel eingesetzt werden, z.B. ein Glasfaserkabel. Die Übertragung des Signals kann jedoch auch kontaktlos erfolgen z.B. als Infrarot-, Radio- oder Funksignal. Es versteht sich, dass in diesem Fall, das von der Vorrichtung erzeugte Signal zunächst von einem Detektor, der ebenfalls in der Vorrichtung oder zumindest in räumlicher Nähe angebracht sein muss, ausgelesen und in ein elektromagnetisches Signal, beispielsweise ein Funksignal, umgewandelt werden muss. Dieses elektromagnetische Signal kann dann von einem außerhalb des Körpers liegenden Empfänger empfangen und ausgewertet werden.

Durch die erfindungsgemäße Verwendung des Hydrogels im Sensor wird eine effiziente ex vivo Diagnostik des Glukosespiegels ermöglicht und damit die Früherkennung von Erkrankungen, die mit gestörtem Glukosemetabolismus einhergehen, sowie deren klinisches Management und die Auswahl von therapeutischen Maßnahmen.

Die Erfindung wird durch die folgenden Ausführungsbeispiele illustriert.

**BEISPIELE**

**Beispiel 1: Herstellung von Sensoren zur Bestimmung von Glukose**

*Herstellung von Hydrogelpartikeln (anreichernde Matrix)*

1 g Alginsäure-Natriumsalz werden in 100 g Wasser unter Rühren gelöst. In einem 5L Becher werden 66,2 g CaC12 x 2H2O in 4931,3 g Wasser gelöst.

Die Alginat-Lösung wird über eine Pumpe in eine Zwei-Stoffdüse gefördert. Gleichzeitig wird am zweiten Eingang der Düse Druckluft angelegt, sodass die Alginat-Lösung in feine Tröpfchen zerstäubt wird. Die Tröpfchen werden durch die Luftströmung in ein Bad mit der Calciumchlorid Lösung getragen, wo sie gelieren und zu Boden sinken. Die gelierten Kugeln werden anschließend gesammelt.

*Herstellung von Sensoren in anreichernden Matrix:*

Alginatkugeln werden zur Beladung nacheinander in einer farbstoffmarkierten ConcanavalinA-Lösung und einer farbstoffmarkierten Dextran-Lösung inkubiert. Anschließend werden die beladenen Kugeln abzentrifugiert und die überstehende Lösung abdekantiert. Die beladenen Kugeln werden ggf. anschließend über Nacht in einer Lösung eines zweiten Polymers (z.B. PVA-, PEGbasiert) inkubiert und ggf. durch Zentrifugation abgetrennt. Anschließend werden die Kugeln in eine wässrige Lösung eines photochemisch vernetzbaren Polymers eingemischt. Diese Mischung wird anschließend mit UV-Licht vernetzt, um das Auslaugen der Sensorkomponenten aus den Alginatkugeln zu verhindern.

Die Mengen richten sich dabei nach der Konzentration des zu messenden Analyten und der Markierungsgrad wird in Abhängigkeit von der gewünschten Intensität des Fluoreszenz-Signals gewählt. Als photochemisch vernetzbares Polymer kann beispielsweise Nelfilcon Polymer, ein mit Acrylamid-Gruppen modifizierter Polyvinylalkohol, verwendet werden. Für die photochemische Vernetzung wird noch 0,1% Irgacure 2959 zugegeben. Die fertige Lösung wird in geeignete Formen dosiert und durch UV-Licht ausgehärtet.

*Herstellung von Sensoren in nicht anreichernder Matrix:*

Farbstoffmarkierte ConcanavalinA-Lösung und farbstoffmarkierte Dextran-Lösung werden nacheinander in eine wasserbasierende Präpolymermischung gegeben und 3 Stunden gerührt.

Die Mengen richten sich dabei nach der Konzentration des zu messenden Analyten und der Markierungsgrad wird in Abhängigkeit von der gewünschten Intensität des Fluoreszenz-Signals gewählt. Als photochemisch vernetzbares Präpolymer kann beispielsweise Nelfilcon Polymer, ein mit Acrylamid-Gruppen modifizierter Polyvinylalkohol, verwendet werden. Für die photochemische Vernetzung wird noch 0,1% Irgacure 2959 zugegeben. Die fertige Lösung wird in geeignete Formen dosiert und durch UV-Licht ausgehärtet.

**Beispiel 2: Bestimmung der Glukoseaktivität für die Sensoren**

*Bestimmung der Glucose-Aktivität in Sensoren:*

Das Fluoreszenzspektrum der Sensoren wird bei verschiedenen Glucosekonzentrationen bestimmt. Die Veränderung der Fluoreszenzintensitäten des Donors mit steigendem Glucosegehalt dient als Maß für die Güte des Glucosesensors. Da bei einem in vivo Glucose-Sensor Glucosekonzentrationen zwischen 50 und 500 mg/dL gemessen werden müssen, wird die Glucose-Aktivität folgendermaßen berechnet:

$$GA = (\text{Intensität}_{500\text{mg/dL}} - \text{Intensität}_{50\text{mg/dL}}) / \text{Intensität}_{50\text{mg/dL}}$$

Alle Responsewerte werden zum besseren Vergleich auf die Response des gleichen Systems in Lösung normiert. Zur Ermittlung der relativen Glucose-Aktivität wird die Glucose-Aktivität der Sensoren (in Matrix) durch die Glucose-Aktivität in Lösung dividiert

$$\text{rel } GA = GA(\text{Matrix}) / GA (\text{Lösung})$$

*Bestimmung der Glucose-Aktivität in Lösung:*

ConA-Lösung und Dextran-Lösung werden in Pufferlösung verdünnt und mehrere Stunden gerührt. Das Fluoreszenzspektrum der Lösung wird bei verschiedenen Glucosekonzen-trationen bestimmt. Die Veränderung der Fluoreszenzintensitäten des Donors mit steigendem Glucosegehalt dient als Maß für die Güte des Systems. Da bei einem in vivo Glucose-Sensor Glucosekonzentrationen zwischen 50 und 500 mg/dL gemessen werden müssen, wird die Glucose-Aktivität folgendermaßen berechnet:

$$GA = (\text{Intensität}_{500\text{mg/dL}} - \text{Intensität}_{50\text{mg/dL}}) / \text{Intensität}_{50\text{mg/dL}}$$

## Beispiel 3: Bestimmung des Einflusses der Matrix

Erfindungsgemäß werden das Glukose-bindende Protein und der Ligand in einer Hydrogelmatrix eingebaut, die eine gewisse Wechselwirkung mit dem Glukose-bindende Protein aufweist. Dabei wird die Hydrogelmatrix so ausgewählt, dass zum einen die Wechselwirkung Glukose-bindendem Protein und Hydrogelmatrix größer ist als die zwischen Glukose-bindendem Protein und wässriger Lösung (Anreicherung der Sensorkomponenten). Andererseits darf aber die Wechselwirkung zwischen Glukose-bindendem Protein und dem Analyten (Glukose) nicht oder nicht wesentlich durch die Wechselwirkung Glukose-bindende Protein und Hydrogelmatrix beeinträchtigt werden.

Bei geeigneter Auswahl der Hydrogelmatrix erzielt man durch die Wechselwirkung zwischen Glukose-bindende Protein und Hydrogelmatrix eine Anreicherung des Glukose-bindenden Proteins in der Matrix weit über die Löslichkeitsgrenze des Glukose-bindende Protein in wässriger Lösung. Im Falle von Con A können beispielsweise bis zu 10-fach höhere Konzentrationen als in freier Lösung erzielt werden. Noch extremer gestalten sich die Löslichkeitsprobleme nach Zugabe des Liganden (z.B. Dextran), da der Glukose-bindende Protein-Ligand - Komplex aufgrund seiner Größe und häufig auch aufgrund von Multivalenzen eine geringere Löslichkeit aufweist. Während der ConA-Dextran-Komplex in Lösung bei einem Massenverhältnis von 1:10 schon ab einer ConA-Konzentration von 0,5 mg/g beginnt auszufallen, können in einer geeigneten Hydrogelmatrix bei gleichem Massenverhältnis ConA-Konzentrationen von über 50 mg/g eingestellt werden. Durch die Hydrogelmatrix kann der anwendbare Konzentrationsbereich des Glucosebindenden Proteins, z.B. ConA, um ein 100-faches erhöht werden.

Da bei einer In vivo-Anwendung der Bereich der Analyt-Konzentration fest liegt und nicht z.B. durch Verdünnen oder Aufkonzentrieren angepasst werden kann, müssen die Konzentrationen von Glukose-bindendem Protein und Ligand auf den In vivo-Konzentrationsbereich des Analyten angepasst werden. Oftmals besteht jedoch die Schwierigkeit, dass deshalb Glukose-bindende Protein-Konzentrationen und / oder Ligand-Konzentrationen nötig wären, die die Löslichkeitsgrenze überschreiten.

Bei dem ConA-Dextran-System kann z.B. in Lösung nur eine ConA-Konzentration von 0,5 mg/g bei einem Massenverhältnis (Dex : ConA) von 1:10 eingestellt werden, da sonst der ConA-Dextran-Komplex beginnt, zu präzipitieren. Die bei dieser Konzentration erreichte Glucose-Aktivität (GA) wird hier zur Ermittlung der relativen Glucose-Aktivität (rel GA) gleich 1 gesetzt. Wie erwartet wird in einer nicht anreichernden Matrix die Glucose-Aktivität aufgrund der geringeren Beweglichkeit der Sensorkomponenten auf die Hälfte verringert (rel GA = 0,52). In anreichernder Matrix wird bei gleicher Konzentration fast die gleiche Response wie in Lösung erhalten (rel GA = 0,83). In der anreichernden Matrix sind jedoch auch wesentlich höhere ConA-Konzentrationen einstellbar. Bei einer ConA-Konzentration von 10 mg/g wird in anreichernder Hydrogelmatrix eine um das 2,6-fache gesteigerte Glucose-Aktivität erhalten (s. Tabelle 1).

Tabelle 1: Einfluss der Matrix

|  | in Lösung | in nicht anreichernder Matrix | in anreichernder Matrix | in anreichernder Matrix |
|---|---|---|---|---|
| ConA-Konzentration | 0,5 mg/g | 0,5 mg/g | 0,5 mg/g | 10 mg/g |
| **rel GA** | **1** | **0,52** | **0,83** | **2,55** |
| Natives ConA mit DOL = 1, Dextran mit 0,001 mol Farbstoff (FS) pro mol Dex-Untereinheit (UE), Massenverhältnis Dex : ConA = 1:10 | | | | |

## Beispiel 4: Bestimmung des Einflusses der Konzentration des Rezeptors

In anreichernder Matrix sieht man deutlich eine Abhängigkeit der Glucose-Aktivität von der Ausgangskonzentration des Glukose-bindende Proteins und des Liganden. Bei einem Anstieg der Konzentration wird auch ein Anstieg der Glucose-Aktivität erhalten. Bei sehr hohen Rezeptor-Konzentrationen nimmt die Glucose-Aktivität wieder ab. Die optimale ConA-Konzentration für einen in vivo-Assay liegt zwischen 8 und 20 mg ConA / g Matrix (s. Tabelle 2).

Tabelle 2: Einfluss der Rezeptor Konzentration

| ConA-Konz [mg/g] | 0,5 | 10,0 | 13,3 | 30 | 52,2 |
|---|---|---|---|---|---|
| **Relative Glucose-Aktivität (rel GA)** | **0,83** | **2,55** | **2,76** | **2,14** | **1,76** |
| Natives ConA mit DOL = 1, Dextran mit 0,001 mol Farbstoff (FS) pro mol Dex-Untereinheit (UE), Massenverhältnis Dex : ConA = 1:10 | | | | | |

**Beispiel 5: Bestimmung des Einflusses des Markierungsgrades Rezeptor**

Aufgrund der mit höheren Wellenlängen abnehmenden Eigenfluoreszenz des Gewebes müssen bei in vivo Anwendungen langwellige Fluoreszenzfarbstoffe eingesetzt werden. Diese Fluoreszenzfarbstoffe sind typischerweise aufgrund der größeren konjugierten Systeme recht unpolar. Wird das Glukose-bindende Protein mit solchen Farbstoffen markiert, so erniedrigt sich seine Löslichkeit, so dass aufgrund des Ausfallens häufig keine hohen Labelgrade möglich sind. Um die Löslichkeit des Glukose-bindende Proteins zu erhöhen, ist es vorteilhaft, diese z.B. mit Polyethylenglycol zu funktionalisieren. Dadurch erhöht sich die Löslichkeit des Glukose-bindende Proteins, wodurch höhere Labelgrade bei der Synthese möglich sind.

Pegylierte ConA's führen jedoch in Lösung nur zu weniger als der Hälfte der Glucose-Aktivität von nativen ConA (rel GA = 0,4). Überraschenderweise tritt in der Hydrogel-Matrix diese Verschlechterung nicht auf. Es wird mit PEG-ConA eine vergleichbare Glucose-Aktivität wie mit nativem ConA erhalten (rel GA = 2,2 vs. 2,6) (s. Tabelle 3).

Tabelle 3: Einfluss des Markierungsgrades (DOL)

| DOL ConA | | 1 | 1 |
|---|---|---|---|
| Typ | | nativ | pegyliert |
| Lösung, c = 0,5 mg ConA / g | rel GA | 1 | 0,43 |
| Matrix, c=10mgConA / g | rel GA | 2,55 | 2,22 |

Durch die Pegylierung wird es erst möglich, ConA mit einem hohen Markierungsgrad eines langwelligen Fluoreszenzfarbstoffes zu versehen, welches in Lösung langzeitstabil ist und nicht präzipitiert. Überraschenderweise wird in anreichernder Matrix im Vergleich zu in Lösung keine Verschlechterung der Glucose-Aktivität durch die Pegylierung beobachtet. Nur durch den positiven Effekt der Matrix ist es somit möglich, in einem Assay PEG-ConA mit hohem Labelgrad in hohen Konzentrationen einzusetzen.

Erstaunlicherweise kann die Glucose-Aktivität durch eine Erhöhung des Labelgrades am ConA zusätzlich gesteigert werden. In der anreichernden Hydrogel-Matrix wird bei ConA mit DOL = 2,5 fast eine Verdoppelung der Glucose-Aktivität erreicht (rel GA = 4,3 vs. 2,6). Im direkten Vergleich zu der Messung in Lösung wird die Glucose-Aktivität in Matrix durch den kombinierten Effekt von Konzentration und DOL somit sogar um das 4,3-fache erhöht (s. Tabelle 4)

Tabelle 4: Einfluss des Markierungsgrades (DOL)

| DOL ConA | | 1 | 1 | 2,5* | 2,5 |
|---|---|---|---|---|---|
| Typ | | nativ | pegyliert | nativ* | Pegyliert |
| Lösung, c = 0,5 mg ConA / g | rel GA | 1 | 0,43 | | 1,03 |
| Matrix, c = 10 mg ConA / g | rel GA | 2,55 | 2,22 | | 4,28 |
| *natives ATTO680-ConA mit einem DOL von >1,5 ist in Lösung nicht stabil und fällt aus. | | | | | |

**Beispiel 6: Bestimmung des Einflusses der Fluoreszenz-Farbstoffe**

Die Struktur der Fluoreszenz-Farbstoffe, die an Glukose-bindendes Protein bzw. Ligand gebunden sind, hat ebenfalls einen Einfluss auf die Glucose-Aktivität. Als besonders geeignet hat sich eine Kombination aus einem Rhodamin- und einem Oxazin-Farbstoff erwiesen. Für das ConA-Dextran-System ist ein Rhodamin-Donor (z.B. ATTO590- oder ATTO610-Dextran, ROX-Dextran) und ein Oxazin-Acceptor (z.B. ATTO655- oder ATTO680-ConA, Evoblue30-ConA) besonders bevorzugt. Im Gegensatz zu dem herkömmlich verwendeten TMR-ConA / FITC-Dextran-System (Xanthen - Rhodamin - Kombination) wird dadurch in Lösung eine 2,1-fache und in anreichernder Matrix sogar eine 2,6-fache Glucose-Aktivität erhalten. Bei einem Rhodamin-Oxazin-Farbstoffpaar kann eine Heterodimer-Wechselwirkung zwischen den Farbstoffen auftreten, welche die Glucose-Aktivität verstärkt (s. Tabelle 5 und 6).

Tabelle 5: Einfluss der Farbstoffe

| ConA-Farbstoff Dextran-Farbstoff | | ATTO680 ATTO590 | TMR FITC |
|---|---|---|---|
| DOL ConA | | 2,5* | 2,3 |
| Typ | | nativ* | nativ |
| Lösung, c = 0,5 mg ConA / g | rel GA | 2,4 | 1,17 |
| Relative Aktivität im Vergleich zu dem herkömmlichen System FITC/TMR | | 2,05 | 1 |
| Matrix, c = 10 mg ConA / g | rel GA | 4,92 | 1,9 |
| Relative Aktivität im Vergleich zu dem herkömmlichen System FITC/TMR | | 2,59 | 1 |
| entsprechenden Werte aufgrund der Erfahrungswerte mit PEG-ConA hochgerechnet: FITC-Dextran mit 0,01 mol FS/mol UE, Massenverhältnis Dex : ConA = 1:10 | | | |

Tabelle 6: Einfluss der Farbstoffe

| | | ATTO590/ATT0680 | ROX/Evoblue30 |
|---|---|---|---|
| Matrix, c = 10 mg ConA / g | rel GA | 2,55 | 2,1 |
| Natives ConA mit DOL = 1, Dextran mit 0,001 mol Farbstoff (FS) pro mol Dex-Untereinheit (UE), Massenverhältnis Dex : ConA = 1:10 | | | |

**Beispiel 7: Bestimmung des Einflusses der Beschaffenheit der Hydrogelmatrix**

Die Glucose-Aktivität ist auch abhängig von der Beschaffenheit der Hydrogel-Matix. Die Hydrogel-Matrix kann aus einem Polymer oder aus einer Mischung von mehreren Polymeren bestehen. Es hat sich eine Hydrogel-Matrix aus Alginat oder einer Mischung aus einem Alginat- und Polyvinylalkohol-Hydrogel als vorteilhaft herausgestellt. Das Alginat-Hydrogel ermöglicht durch seine Wechselwirkung mit dem ConA und dem Dextran die Anreicherung der Sensorkomponenten in hohen Konzentrationen.

Das Präpolymer des 2. Hydrogels (z.B. PVA) kann in die Alginatkugeln eindringen und nach seiner Vernetzung ein interpenetrating Network mit dem Alginat bilden. Die Maschenweite des Interpenetrating Network beeinflusst die Beweglichkeit der Moleküle des Glukose-bindenden Proteins und des Liganden und somit auch die Glucose-Aktivität. Die Maschenweite kann beeinflusst werden durch die Art, das Molekulargewicht, den Feststoffgehalt des Polymers sowie den Anteil an Vernetzergruppen, welcher die Anzahl an Knotenpunkten bestimmt.

Ein geringerer Gehalt an Vernetzergruppen führt zu einer höheren Glucose-Aktivität. Durch eine Halbierung der Vernetzergruppen kann die Aktivität sogar um das 1,5-fache gesteigert werden. Im Vergleich zu dem System in Lösung kann somit ebenfalls eine 4,2-fache Verbesserung der Glucose-Aktivität erzielt werden, ohne eine Erhöhung des Labelgrades am ConA (s. Tabelle 7).

Tabelle 7: Einfluss des Vernetzergehaltes

| **Vernetzergehalt (mmol/g)** | **0,486** | **0,33** | **0,26** |
|---|---|---|---|
| rel GA | 2,76 | 4,04 | 4,24 |
| Natives ConA (13 mg/g) mit DOL = 1, Dextran mit 0,001 mol Farbstoff (FS) pro mol Dex-Untereinheit (UE), Massenverhältnis Dex : ConA = 1:10 | | | |

Der Feststoffgehalt des Netzwerkes hat ebenfalls einen Einfluss auf die Glucose-Aktivität. Je höher der Feststoffgehalt umso geringer ist die Glucose-Aktivität (s. Tabelle 8).

Tabelle 8: Einfluss des Feststoffgehaltes

| FG Präpolymer | 25% | 30% | 35% | 40% |
|---|---|---|---|---|
| rel. GA | 2,87 | 2,76 | 2,48 | 1,96 |
| Natives ConA (13 mg/g) mit DOL = 1, Dextran mit 0,001 mol Farbstoff (FS) pro mol Dex-Untereinheit (UE), Massenverhältnis Dex : ConA = 1:10 | | | | |

**Patentansprüche**

1. Verwendung eines Polymer-basierten Hydrogels bestehend aus zumindest einem wasserlöslichen Polymer in einem Sensor zum Anreichern eines gelösten Glukose-bindenden Proteins, wobei das zumindest eine Polymer in der Lage ist, mit dem Glukose-bindenden Protein zu interagieren und wobei das Glukose-bindende Protein in einem Komplex mit einem Liganden vorliegt, wobei das Polymer-basierte Hydrogel eine erste Hydrogel-matrix aus Alginat umfasst und eine zweite Hydrogelmatrix, die innerhalb der ersten Hydrogelmatrix ein interpenetrierendes Netzwerk ausbildet.

2. Verwendung nach Anspruch 1, wobei der Ligand des Glukose-bindenden Proteins ein Oligosaccharid, ein glykosyliertes Markromolekül oder ein glykosylierter Nanopartikel ist.

3. Verwendung nach einem der Ansprüche 1 oder 2, wobei das gelöste Glukose-bindende Protein in einer im Vergleich zu einer wässrigen Lösung mindestens 5-fach, mindestens 10-fach, mindestens 15-fach oder mindestens 20-fach erhöhte Konzentration vorliegt.

4. Verwendung nach einem der Ansprüche 1 bis 3, wobei das Glukose-bindende Protein ausgewählt ist aus der Gruppe bestehend aus: Lektinen, Enzymen, die Glukose als Substrat binden, Antikörpern, die spezifisch Glukose erkennen, und Aptameren, die spezifisch Glukose erkennen.

5. Verwendung nach einem der Ansprüche 1 bis 4, wobei das Glukose-bindende Protein Concanavalin A ist.

6. Verwendung nach Anspruch 5, wobei die Concanavalin A-Konzentration größer als 0,5 mg/(g Polymer) ist.

7. Verwendung nach Anspruch 6, wobei die Concanavalin A-Konzentration zwischen 2 und 60 mg/(g Polymer) liegt.

8. Verwendung nach einem der Ansprüche 5 bis 7, wobei das Concanavalin A durch chemische Modifikation eine im Vergleich zu unmodifiziertem Concanavalin A erhöhte Wasserlöslichkeit aufweist.

9. Verwendung nach Anspruch 8, wobei die Modifikation ausgewählt ist aus der Gruppe bestehend aus: Pegylierung, Acetylierung, Succinylierung und Polyoxazolinylierung.

10. Verwendung nach einem der Ansprüche 2 bis 9, wobei das Glukose-bindende Protein mit einem Oxazin-Farbstoff verknüpft und der Ligand des Glukose-bindenden Proteins mit einem Rhodamin-Farbstoff verknüpft ist.

11. Verwendung nach einem der Ansprüche 1 bis 10, wobei der Sensor zur in vitro Bestimmung des Glukosegehalts eingesetzt wird.

12. Verwendung nach einem der Ansprüche 1 bis 10, wobei der Sensor zur in vitro Diagnose eines gestörten Glukosemetabolismus eingesetzt wird.

13. Verwendung nach einem der Ansprüche 1 bis 10, wobei der Sensor für die Bestimmung der Notwendigkeit einer therapeutischen Maßnahme bei einem Patienten mit gestörtem Glukosemetabolismus eingesetzt wird.

**Claims**

1. Use of a polymer-based hydrogel consisting of at least one water-soluble polymer in a sensor for the concentration of a dissolved glucose-binding protein, where the at least one polymer is able to interact with the glucose-binding protein and where the glucose-binding protein is present in a complex with a ligand, where the polymer-based hydrogel comprises a first hydrogel matrix of alginate and a second hydrogel matrix which forms an interpenetrating network within the first hydrogel matrix.

2. Use according to Claim 1, where the ligand of the glucose-binding protein is an oligosaccharide, a glycosylated macromolecule or a glycosylated nanoparticle.

3. Use according to one of Claims 1 or 2, where the dissolved glucose-binding protein is present in a concentration increased at least 5-fold, at least 10-fold, at least 15-fold or at least 20-fold, compared to an aqueous solution.

4. Use according to one of Claims 1 to 3, where the glucose-binding protein is selected from the group consisting of: lectins, enzymes which bind glucose as substrate, antibodies which specifically recognize glucose, and aptamers which specifically recognize glucose.

5. Use according to one of Claims 1 to 4, where the glucose-binding protein is concanavalin A.

6. Use according to Claim 5, where the concanavalin A concentration is greater than 0.5 mg/(g of polymer).

7. Use according to Claim 6, where the concanavalin A concentration is between 2 and 60 mg/(g of polymer).

8. Use according to one of Claims 5 to 7, where the concanavalin A has an increased solubility in water compared to unmodified concanavalin A as a result of chemical modification.

9. Use according to Claim 8, where the modification is selected from the group consisting of: pegylation, acetylation, succinylation and polyoxazolinylation.

10. Use according to one of Claims 2 to 9, where the glucose-binding protein is linked with an oxazine dye and the ligand of the glucose-binding protein is linked with a rhodamine dye.

11. Use according to one of Claims 1 to 10, where the sensor is used for the in vitro determination of glucose content.

12. Use according to one of Claims 1 to 10, where the sensor is used for the in vitro diagnosis of a disturbed glucose metabolism.

13. Use according to one of Claims 1 to 10, where the sensor is used for determining the need for a therapeutic measure in a patient with disturbed glucose metabolism.

**Revendications**

1. Utilisation d'un hydrogel à base de polymère, constitué d'au moins un polymère hydrosoluble, dans un capteur pour la concentration d'une protéine se liant au glucose dissous, ledit au moins un polymère étant capable d'interagir avec la protéine se liant au glucose et la protéine se liant au glucose étant présente en un complexe avec un ligand, l'hydrogel à base de polymère comprenant une première matrice d'hydrogel à base d'alginate et une deuxième matrice d'hydrogel, qui forme à l'intérieur de la première matrice d'hydrogel un réseau interpénétrant.

2. Utilisation selon la revendication 1, dans laquelle le ligand de la protéine se liant au glucose est un oligosaccharide, une macromolécule glycosylée ou une nanoparticule glycosylée.

3. Utilisation selon l'une quelconque des revendications 1 et 2, dans laquelle la protéine se liant au glucose dissous est présente à une concentration augmentée d'au moins 5 fois, d'au moins 10 fois, d'au moins 15 fois ou d'au moins 20 fois en comparaison d'une solution aqueuse.

4. Utilisation selon l'une quelconque des revendications 1 à 3, dans laquelle la protéine se liant au glucose est choisie dans le groupe constitué par : des lectines, des enzymes qui se lient au glucose en tant que substrat, des anticorps qui reconnaissent spécifiquement le glucose, et des aptamères qui reconnaissent spécifiquement le glucose.

5. Utilisation selon l'une quelconque des revendications 1 à 4, dans laquelle la protéine se liant au glucose est la concanavaline A.

6. Utilisation selon la revendication 5, dans laquelle la concentration de la concanavaline A est supérieure à 0,5 mg/(g de polymère).

7. Utilisation selon la revendication 6, dans laquelle la concentration de la concanavaline A est comprise entre 2 et 60 mg/(g de polymère).

8. Utilisation selon l'une quelconque des revendications 5 à 7, dans laquelle la concanavaline A présente par modification chimique une solubilité dans l'eau qui est accrue en comparaison de la concanavaline A non modifiée.

9. Utilisation selon la revendication 8, dans laquelle la modification est choisie dans le groupe constitué par : la pégylation, l'acétylation, la succinylation et la polyoxazolinylation.

10. Utilisation selon l'une quelconque des revendications 2 à 9, dans laquelle la protéine se liant au glucose est attachée à un colorant oxazine et le ligand de la protéine se liant au glucose est attaché à un colorant rhodamine.

11. Utilisation selon l'une quelconque des revendications 1 à 10, dans laquelle on utilise le capteur pour la détermination in vitro de la teneur en glucose.

12. Utilisation selon l'une quelconque des revendications 1 à 10, dans laquelle on utilise le capteur pour le diagnostic in vitro d'un métabolisme perturbé du glucose.

13. Utilisation selon l'une quelconque des revendications 1 à 10, dans laquelle on utilise le capteur pour la détermination de la nécessité d'une intervention thérapeutique chez un patient à métabolisme perturbé du glucose.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070122829 A
- US 6485703 B
- US 20070105176 A
- WO 200113783 A
- WO 2002087429 A
- US 20070105176 A1

**In der Beschreibung aufgeführte Nicht-Patentliteratur**

- **ROUNDS.** *J. Fluorec.,* 2007, vol. 17, 57-63
- **CHEUNG et al.** *Anal. Chim. Acta,* 2008, vol. 607, 204-210
- **MOOTHOO.** *Glycobiology,* 1999, vol. 9 (6), 539-545
- **BRYCE.** *Biophysical Journal,* 2001, vol. 81, 1373-1388
- **MOOTHOO.** *Glycobiology,* 1998, vol. 8 (2), 173-181
- **DELATORRE.** *BMC Structural Biology,* 2007, vol. 7, 52
- **YAMAUCHI.** *FEBS Letters,* 1990, vol. 260 (1), 127-130
- **BIRCH.** *Spectrochimica Acta,* 2001, vol. 57, 2245-2254
- **ROUNDS.** *J. Fluoresc.,* 2007, vol. 17, 57-63